(19) Europäisches Patentamt
European Patent Office
Office européen des brevets

(11) Publication number: **0 029 341**
**B1**

(12) **EUROPEAN PATENT SPECIFICATION**

(45) Date of publication of patent specification: **22.02.84**

(51) Int. Cl.³: **C 07 D 209/52, A 61 K 31/40**

(21) Application number: **80304052.6**

(22) Date of filing: **12.11.80**

(54) **Nitrogen-containing prostacyclin analogues, their preparation and compositions containing them.**

(30) Priority: **15.11.79 US 94572**
**16.06.80 US 159738**

(43) Date of publication of application:
**27.05.81 Bulletin 81/21**

(45) Publication of the grant of the patent:
**22.02.84 Bulletin 84/8**

(84) Designated Contracting States:
**BE CH DE FR GB IT LI NL SE**

(56) References cited:
**EP - A - 0 015 227**
**DE - A - 2 909 361**
**DE - A - 2 940 746**
**US - A - 4 097 489**

**TETRAHEDRON LETTERS, Nr. 16, April 1978, G.L. BUNDY et al. "The Synthesis of Nitrogen-Containing Prostacyclin Analogs" pages 1371 to 1374**

(73) Proprietor: **THE UPJOHN COMPANY**
**301 Henrietta Street**
**Kalamazoo, Michigan 49001 (US)**

(72) Inventor: **Smith, Herman Walden**
**806 Boswell Lane**
**Kalamazoo Michigan (US)**

(74) Representative: **Perry, Robert Edward et al,**
**GILL JENNINGS & EVERY 53-64 Chancery Lane**
**London WC2A 1HN (GB)**

**EP 0 029 341 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Nitrogen-containing prostacyclin analogues, their preparation and compositions containing them

This invention relates to novel nitrogen-containing prostacyclin analogues, to methods for preparing them and to compositions containing them. The compounds have been found to have pharmaceutical utility.

Prostacyclin is an endogenously produced compound in mammalian species, being structurally and bio-synthetically related to the prostaglandins. Nicolaou *et al,* J.A.C.S. *101 (3)* (1979) 766 disclose, 6,9-pyridazaprostacyclin and its derivatives dihydropyridazaprostacyclin and N-oxides thereof. U.S. Patent Specification No. 4,112,224 discloses bicyclic nitrogen-containing prostaglandin analogues.

DE-A-2 909 361 and 2 940 746, EP-A-15227, US-A-4,097,489 and Bundy *et al,* Tet. Letters *16* (1978) 1371-1374, disclose 9-deoxy-9$\alpha$,6-nitrilo-PGF or 6,9$\alpha$-imino-PGF compounds.

The novel compounds of this invention are of formula I

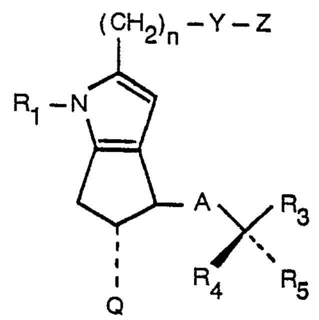

I

wherein Q is hydrogen, OH or $CH_2OH$;

*n* is 2 or 4;

Y is $-CH_2CH_2-$, *trans*-$CH{=}CH-$ or $-CH_2CF_2$;

Z is $COOR_8$, $CONR_{13}R_{14}$ or $CH_2OR_9$ in which $R_8$ is selected from hydrogen, $C_{1-6}$ alkyl, a pharmacologically acceptable metal or amine cation, phenyl, phenyl substituted by up to 3 chlorine atoms or $C_{1-3}$ alkyl radicals and *p*-substituted phenyl in which the substituent is $NHCOR_{10}$, $COR_{11}$, $OCOR_{12}$ or $CH{=}N-NH-CONH_2$ in which $R_{10}$ is methyl, phenyl, acetamidophenyl, benzamidophenyl or $NH_2$, $R_{11}$ is methyl, phenyl, $NH_2$ or methoxy and $R_{12}$ is phenyl or acetamidophenyl, $R_{13}$ and $R_{14}$ are independently selected from hydrogen, $C_{1-6}$ alkyl, phenyl and benzyl, and $R_9$ is selected from hydrogen, $C_{1-6}$ alkyl, benzoyl and acetyl;

$R_1$ is hydrogen, $C_{1-4}$ alkyl, $C_{3-7}$ cycloalkyl, phenyl, phenyl substituted by up to 3 substituents selected from $C_{1-3}$ alkyl, fluorine, chlorine, trifluoromethyl and methoxy, phenyl substituted by $COOR_8$ in which $R_8$ is as defined above, phenyl substituted by $CH_2OR_9$ in which $R_9$ is as defined above, or biphenylyl;

A is $-CH_2CH_2-$, *cis*-$CH{=}CH-$, *trans*-$CH{=}CH-$ or $-C{\equiv}C-$;

either one of $R_4$ and $R_5$ is hydrogen or $C_{1-3}$ alkyl and the other is hydroxy, methoxy or acetoxy or $R_4$ and $R_5$ are both hydrogen or $R_4$ and $R_5$ taken together are oxo; and

$R_3$ is $CR_6R_7-X_1-CH_3$, $CR_6R_7-X_2-E$ or *cis*-$CH_2-CH{=}CH-CH_2-CH_3$ in which $X_1$ is $C_{1-4}$ alkylene, $X_2$ is a valence bond, O or $C_{1-6}$ alkylene, $R_6$ and $R_7$ are independently selected from hydrogen, fluorine and $C_{1-4}$ alkyl, provided that $CR_6R_7$ is not CFAlkyl and that neither $R_6$ nor $R_7$ is fluorine when $X_2$ is O, and E is phenyl, phenyl substituted by up to 3 substituents independently selected from $C_{1-3}$ alkyl, fluorine, chlorien, trifluoromethyl and methoxy, phenyl substituted by $COOR_8$ in which $R_8$ is as defined above, phenyl substituted by $CH_2OR_9$ in which $R_9$ is as defined above, or biphenylyl.

A preferred group of compounds of the invention are those of formula I wherein Y is $-CH_2CH_2-$ or $-CH_2CF_2-$;

Z is $COOR_8$ in which $R_8$ is hydrogen, methyl or a pharmacologically acceptable metal or amine cation;

$R_1$ is hydrogen, $C_{1-4}$ alkyl, cyclohexyl or phenyl;

A is $-CH_2CH_2-$, *trans*-$CH{=}CH-$ or $-C{\equiv}C-$;

either one of $R_4$ and $R_5$ is hydrogen or methyl and the other is hydroxy or $R_4$ and $R_5$ are each hydrogen or $R_4$ and $R_5$ taken together are oxo;

$R_6$ and $R_7$ when $R_3$ is $CR_6R_7-X_1-CH_3$, are each fluorine or methyl and, when $R_3$ is $CR_6R_7-X_2-E$, are each hydrogen or methyl; and

E, if present, is phenyl optionally substituted by one or two substituents independently selected from fluorine, chlorine, trifluoromethyl and methoxy.

A more preferred group of compounds of the invention are those of formula I in which Q is OH; *n* is 2; Y is $-CH_2CH_2-$ or $-CH_2CF_2$; Z is COOH or $COOCH_3$; $R_1$ is hydrogen, methyl or phenyl; A is *trans*-

CH=CH— or —C≡C—; $R_4$ is hydrogen; $R_5$ is hydroxy; and $R_3$ is ($C_4$ alkyl)methyl.

An especially preferred group of compounds of the invention are those of formula I in which Q is OH; *n* is 2; Y is *trans*-CH=CH—; Z is COOH, $COOCH_3$ or $CONHR_{14}$ in $R_{14}$ is hydrogen, methyl or phenyl; $R_1$ is hydrogen or methyl; A is *trans*—CH=cH—; $R_4$ is methyl; $R_5$ is hydroxy; and $R_3$ is ($C_4$ alkyl)methyl.

Another especially preferred group of compounds of the invention are those of formula I in which Q is OH; *n* is 2; Y is —$CH_2CH_2$—; Z is COOH; $R_1$ is phenyl; A is *trans*-CH=CH—; $R_4$ is hydrogen; $R_5$ is hydroxy; and $R_3$ is 1,1-dimethyl-($C_4$ alkyl)methyl, phenethyl or phenoxymethyl.

Examples of particularly preferred compounds of this invention are 9-deoxy-6,9-N-phenylimino-$\Delta^{6,8}$-prostacyclin, its 13,14-dihydro analogue, and their methyl esters.

The stereochemistry of the compounds of this invention is represented in the formulae herein in the usual convention for prostacyclins. The nomenclature used herein is also that conventionally used for prostacyclins; see Nelson, Med. Chem. *17* (1974) 911 and Johnson, Prostaglandins *15* (1978) 737.

Unless otherwise specified, alkyl groups discussed herein may be straight or branched. Thus "$C_{1-6}$ alkyl" means methyl, ethyl, propyl, butyl, pentyl, hexyl and the isomers thereof, for example isopropyl, *tert*-butyl, neopentyl and 2,3-dimethylbutyl. "$C_{3-7}$ cycloalkyl" means cyclopropyl, cyclobutyl, cyclopnetyl, cyclohexyl or cycloheptyl.

The phrase "pharmacologically acceptable amine salt" refers to salts derived from amines which are essentially non-toxic when administered to mammals. The amine may be a primary, secondary or tertiary amine. Examples of suitable amines are methylamine, dimethylamine, triethylamine, ethylamine, dibutylamine, triisopropylamine, N-methylhexylamine, decylamine, dodecylamine, allylamine, crotylamine, cyclopentylamine, dicylohexylamine, benzylamine, dibenzylamine, α-phenylethylamine, β-phenylethylamine, ethylenediamine, diethylenetriamine, adamantylamines, and like aliphatic, cycloaliphatic and araliphatic amines containing up to 18 carbon atoms, as well as heterocyclic amines, e.g. piperidine, morpholine, pyrrolidine, piperazine, and lower alkyl derivatives thereof, e.g. 1-methylpiperidine, 4-ethylamorpholine, 1-isopropylpyrrolidine, 2-methylpyrrolidine, 1,4-dimethylpiperazine and 2-methylpiperidine, as well as amines containing water-solubilizing or hydrophilic groups, e.g., mono-, di- and triethanolamine, ethyldiethanolamine, N-butylethanolamine, 2-amino-1-butanol, 2-amino-1-ethyl-1,3-propanediol, 2-amino-2-methyl-1-propanal, tri(hydroxymethyl)aminoethane, N-phenylethanolamine, N-(*p*-*tert*-amylphenyl) diethanolamine, galactamine, N-methylglucamine, N-methylglucosamine, ephedrine, phenylephrine, epinephrine and procaine.

The term "pharmacologically acceptable metal" includes alkali metals such as sodium and potassium, alkaline earth metals such as calcium and magnesium, and other acceptable metals such as aluminium.

In the case when Z is $COOR_8$ and $R_8$ is *p*-substituted phenyl, examples of such esterifying groups include *p*-acetamidophenyl, *p*-benzamindophenyl, *p*-(*p*-acetamidobenzamido)phenyl, *p*-(*p*-benzamidobenzamido) phenyl, *p*-amidocarbonylaminophenyl, *p*-acetylphenyl, *p*-benzylphenyl, *p*-amidocarbonylphenyl, *p*-methoxycarbonylphenyl, *p*-benzoyloxyphenyl and *p*-(*p*-actamidobenzoyloxy)phenyl.

The compounds of formula I wherein Z is COOH, and hydroxy-protected analogues thereof, may be prepared by reacting a compound of formula V

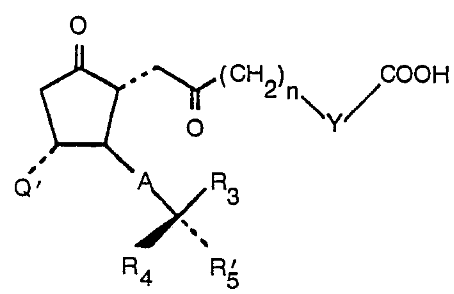

V

wherein Q′ and $R_5'$ are as defined for Q and $R_5$ above but may also be protected OH, and *n*, Y, A, $R_3$ and $R_4$ are as defined above, with an amine of the formula

$$R_1NH_2$$

wherein $R_1$ is as defined above, in an organic solvent.

Many of the 6-oxo-PGE compounds of formula V are described and claimed in GB-A-1,565,604. The compounds of formula V may be prepared by the sequence shown in the following chart.

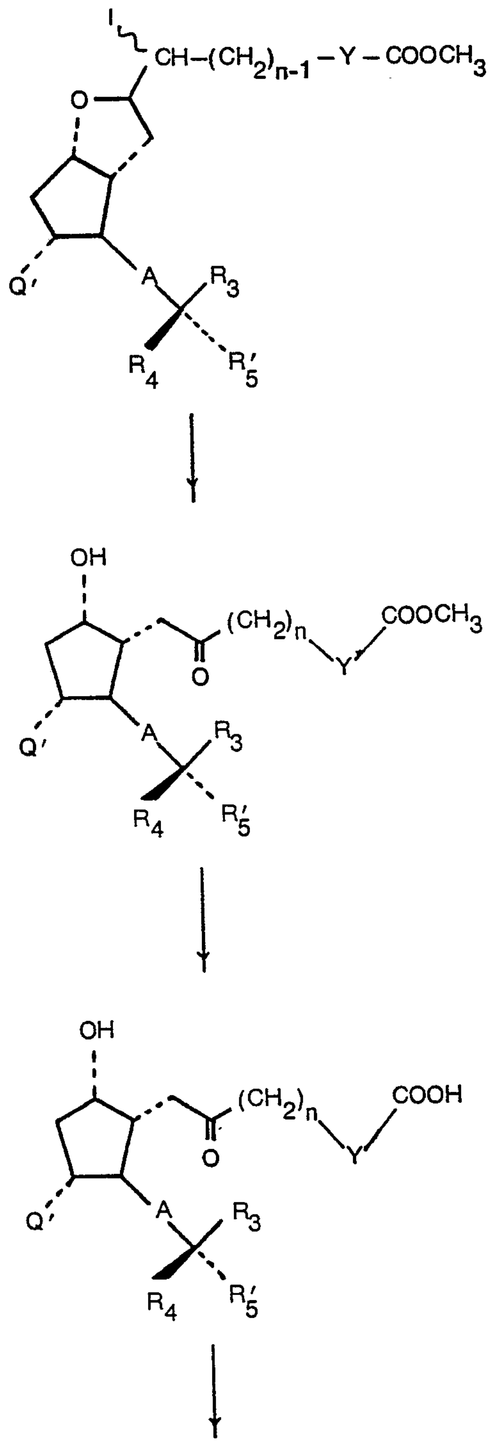

II

III

IV

V

The compounds of formula II are known or can be prepared by conventional procedures from known compounds. For example, the given methyl esters can be prepared from the corresponding acids by known esterification procedures.

It is generally preferred that Q′ and $R_5'$ should both be protected OH. Suitable blocking groups may be introduced into the corresponding unblocked compounds by conventional procedures before the sequence of reactions shown in the chart is conducted. Suitable blocking groups, and methods for their introduction and removal, are disclosed in British Patent Specification No. 1,565,604. An example is 2-tetrahydrofuanyl. Silyl blocking groups are also suitable.

In carrying out the sequence of reactions shown in the above chart, with subsequent conversion

of a formula V compound to a formula I compound, it is usually preferred to remove any blocking group immediately before the compound of formula I is prepared. Alternatively, the blocking group may be removed subsequently.

A compound of formula II may be converted to a compound of formula III by dehydroidination and hydrolysis in the same manner as is disclosed for the conversion of compounds VIII to IV in British Patent Specification No. 1,565,604.

A compound of formula III may be converted to a compound of formula IV by saponification. One method of achieving saponification is to replace the 9-hydroxy group by a 9-acetoxy group, suitably by reaction of the formula III compound with an acetylating agent such as acetic anhydride, suitably in a solvent. Hydrolysis of the 9-acetate, e.g. with sodium hydroxide, causes saponification and removal of the acetyl group, giving a compound of formula IV.

A compound of formula IV may be converted to a compound of formula V by oxidation in the same manner as is disclosed for the conversion of compounds IV to V in British Patent Specification No. 1,565,604.

It may be possible to form a compound of formula V in methyl ester form by direct oxidation of a compound of formula III.

A compound of formula V might be prepared by dehydroidination and hydrolysis of a compound of formula II in acid form to a compound of formula IV, followed by oxidation.

Whether it is desired to remove any hydroxy protective group once the formula V compound has been prepared, or after it has been converted to a compound of formula I, this can be achieved by standard methods. For example, acidic hydrolysis may be used to remove a tetrahydropyranyl group, and contact with an anionic fluoride-containing compound may be used to remove a silyl ether.

The conversion of a 6-oxo-PGE compound of formula V to a pyrrole-type compound of formula I is carried out in a suitable solvent such as a lower alcohol, ether or amide. Examples of suitable lower alcohols are methanol, ethanol, propanol and isopropanol. Examples of suitable ethers are diethyl ether, dioxane and tetrahydrofuran. Examples of suitable amides are dimethylformamide and diethylformamide. The temperature at which the reaction is conducted is not unduly critical but is conveniently from 25 to 50°C, depending upon the reaction velocity desired. Catalytic quantities of an acid, e.g. a mineral acid such as hydrochoric acid or sulphuric acid, an amine hydrochloride or an organic sulphonic acid such as *p*-toluenesulphonic acid are generally employed.

A compound of formula I in which Z is COOH or $COOCH_3$ may be converted to any other compound of formula I in which Z is as defined above by conventional procedures.

Suitably by the methods described above, compounds of formula I and V may be prepared having the structural features disclosed at from line 60 on page 22 to line 31 on page 25 of GB-A-1,565,604, as well as the 13-*trans* isomers of the given 13-*cis* groups, and also the following:

15-methyl-;
2,3-didehydro-;
*trans*-2,3-dehydro-;
15-oxo-2,3-difluoro-;
15-dihydro-;
15-methoxy-;
15-methoxy-13,14-dihydro-;
15-acetyl-;
15-methoxy-;
15-acetyl-17-phenoxy-18,19,20-trinor-;
15-actyle-13-*trans*-;
20-methyl-;
20-nor-;
19,20-dinor-;
15-ethyl-18,19,20-trinor-;
15-methyl-18,19,20-trinor-;
13,14-didehydro-;
2-*trans*-20-nor-;
15-ethyl-15-methoxy-18,19,20-trinor-;
16,16-difluoro-ethylenyl-19-phenyl-20-nor-;
18-(*p*-chlorophenyl)-20-nor-;
*cis*-17,18-didehydro-;
2,2-difluoro-*cis*-17,18-didehydro-;
13,14-dihydro-*cis*-17,18-didehydro-;
*trans*-2-*cis*-17,18-didehydro-;
1a, 1b-dihomo-*cis*-17,18-didehydro-; and
*trans*-2-3a,3b-dihomo-*trans*-13,14-didehydro-16-phenoxy-18,19,20-trinor-.

The following Examples illustrate how the compounds of the invention may be prepared. All solvent proportions are by volume.

Example 1

9-Deoxy-6,9-N-phenylimino-$\Delta^{6,8}$-PGI methyl ester

1a. *5ξ-Iodo-9-deoxy-6ξ,9α-epoxy-PGF$_{1\alpha}$11,15-bis (dimethyl-t-butylsilyl) ether methyl ester*

A solution of 5ξ-iodo-9-deoxy-6ξ,9α-epoxy-PGF$_{1\alpha}$ methyl ester (11.0 g) in DMF (50 ml) is treated with imidazole (8.86 g) and t-butyldimethylchlorosilane (7.85 g) and reacted for two hours. The suspension is diluted with $H_2O$ and extracted with ethyl acetate. Drying and evaporation of the extract gives 15 g of a pale yellow oil. Purification on silica gel (360 g) with 97:3 hexane:ethyl acetate (260 ml fractions) gives 10.9 g of 1*a*.

1b. *6-Keto-PGF$_{1\alpha}$ bis(dimethyl-t-butylsilyl) ester methyl ester*

A solution of 1*a* (10.9 g) in toluene (50 ml) is treated with diazabicyclononene (12.5 ml) and reacted for 19 hours. Additional reagent (8.2 ml) is added and the suspension reacted for six hours. The reaction is diluted with ethyl acetate, partitioned with $H_2O$, dried and evaporated to a green oil.

The crude oil is hydrolysed in acetonitrile (5 ml) with 4% acetic acid in 90% aqueous acetonitrile for 45 minutes, diluted with 0.2 M $KHSO_4$, extracted with ether, and the combined extracts washed with $H_2O$. Drying and evaporation of solvent gives a two-product mixture (8.96 g).

Purification on silica gel (440 g) with 85:15 hexane:ehtyl acetate (40 ml fractions) gives 1.65 g of impure hemi-acetal; 7:3 hexane:ethyl acetate elution (fractions 10-43) gives 7.1 g of 1*b* containing 25-30% of the hemiacetal, Rf 0.40 and 0.77, respectively in 3:1 cyclohexane; ethyl acetate; Rf 0.08 and 0.31, respectively, in 9:1 cyclohexane:ethyl acetate.

1c. *6-Keto-PGF$_{1\alpha}$ 9-acetate 11,15(bis-t-butyl-dimethylsilyl) ether methyl ester*

A solution of crude 1*b* (5.1 g) in pyridine (20 ml) is treated with acetic anhydride and reacted for 65 hours. The solution is cooled, diluted with 5% NaCl and extracted with ether. The extract is washed, dried and evaporated to yield crude 1*c* as a pale yellow oil (5.78 g).

Fractionation on silica gel (425 g) with 9:1 hexane:ethyl acetate (fractions 1-19) and 8.5:1.5 hexane:ethyl acetate (fractions 20-46) gives 1c (3.83 g) in fractions 21-29.

1d. *6-Keto-PGF$_{1\alpha}$ 11,15-bis(t-butyldimethylsilyl) ether*

A solution of 1*c* (1.43 g) in dimethylformamide (90 ml) is treated with 2.2N NaOH (10 ml) and the turbid mixture treated with $H_2O$ (12 ml). The solution is heated at 80-87°C for 1.5 hours, cooled, acidified with 0.2 M $KHSO_4$ and extracted with ethyl acetate. The extract is washed, dried and evaporated to yield 1*d* (1.01 g) as a pale yellow oil, Rf 0.27 in solvent 2:1 cyclohexane; Organic layer from 9:2:5:10 ethyl acetate:acetic acid:cyclohexane:water. Neat storage at 4°C produces an impurity (*ca.* 20%) at Rf 0.34.

The sample is combined with product (2.8 g) from duplicate hydrolysis and purified on CC-4 silica gel (430 g) with 9:1 hexane:ethyl acetate collecting 48 ml fractions for fractions 1-84 and 8.5:1.5 for fractions 85-168 (55 ml fractions). Fractions 130-168 provide 2.58 g (61%) of 1*d.*

1e. *6-Keto-PGE$_1$ 11,15-bis(t-butyldimethylsilyl) ether*

A solution of 1*d* (2.5 g) in acetone (45 ml) at −40°C is treated with 2.67 M Jones reagent (3.25 ml), brought to −20°C during eight minutes and reacted at −20°C for 75 minutes. The suspension is diluted with cold 5% NaCl, extracted with ether and the combined extracts are washed with 5% NaCl. Drying and evaporation of solvent gives 2.0 g of yellow oil. Fractionation on CC-4 silica gel (210 g) with 8.5:1.5 hexane:ethyl acetate (50 ml fractions) removes polar impurities. Fractions 23-50 give pure 1*e* (0.85 g), Rf 0.25 in 2:1 cyclohexane:organic layer from 9:2:5:10 ethyl acetate:acetic acid:cyclohexane:water.

1f. *9-Deoxy-6,9-N-phenylimino-$\Delta^{6,8}$-PGI$_1$ 11,15-bis(t-butyldimethylsilyl) ether methyl ester*

A solution of 1*e* (0.85 g) in ethanol (2 ml) is treated with 1 M aniline in ethanol (10 ml) and 0.1 M pyridine hydrochloride in methylene chloride (0.08 ml) and reacted for 26 hours. The solution is diluted with ether, washed with 0.2 M $KHSO_4$ and 5% NaCl solutions, dried and evaporated to yield crude 1*f*. Without purification, the prepared acid is esterified with excess ethereal diazomethane to yield crude 1*f* (0.90 g).

Fractionation on CC-4 silica gel with 95:5 hexane:ethyl acetate (18 ml fractions) gives pure 1*f* (0.52 g), Rf 0.53, 0.66 in 2:1 cyclohexane: organic layer from 9:2:5:10 ethyl acetate:acetic acid:cyclohexane:water, and (9:1 cyclohexane:ehtyl acetate, respectively in fractions 6-13. Pure acid (0.116 g) is obtained in fractions 29-63 (Rf 0.45 in 2:1 cyclohexane:organic layer from 9:2:5:10 ethyl acetate:acetic acid:cyclohexane:water.

1g. *9-Deoxy-6,9-N-phenylimino-$\Delta^{6,8}$-PGI$_1$ methyl ester*

A solution of 1*f* (0.51 g) in tetrahydrofuran (THF) is treated with 1.2 M tetrabutylammonium fluoride in THF (5 ml) and reacted for 1.5 hours. The dark solution is diluted with ether, washed with 0.2 M $KHSO_4$ and 5% NaCl solutions, dried and evaporated. The residue (0.42 g) is purified on CC-4 silica gel (60 g), colllecting 17 ml fractions. Fractions 95-120 (3:2 hexane:ethyl acetate) provide 78 mg

of 1*g* (product compound), m.p. 95.5°C after ether-hexane crystallization.

IR (mull) 965, 1060, 1495, 1605, 1745, 2975, 3425 $cm^{-1}$.

Ultraviolet (Ethanol) 233 ($\varepsilon$ 11,400) $\eta$m.

Example 2

9-Deoxy-6,9-N-phenylimino-$\Delta^{6,8}$-$PGI_1$

A solution of 9-deoxy-6,9-N-phenylimino-$\Delta^{6,8}$-$PGI_1$ methyl ester (0.320 g) in methanol (20 ml) and 1N sodium hydroxide (4 ml) is reacted at 25°C for 18 hours. The solution is acidified with 1M citric acid and the precipitate is collected to yield 0.274 g of the product compound. Recrystallization from methylene chloride-hexane gives the product compound, m.p. 140.5°C.

Example 3

9-Deoxy-6,9-N-phenylimino-$\Delta^{6,8}$-13,14-dihydo-$PGI_1$

3a. *9-Deoxy-6,9-N-phenylimino-$\Delta^{6,8}$-13,14-dihydro-$PGI_1$ methyl ester di (etrahydropyranyl) ether*

A solution of 13,14-dihydro-6-keto-$PGE_1$ ditetrahydropyranyl ether (0.31 g) in ethanol (0.5 ml) is treated with 1 M aniline in ethanol (4.0 ml) and 0.1 M pyridine hydrochloride in $CH_2Cl_2$ (0.05 ml). The solution is reacted at 25°C for 7 hours, diluted with ether and washed with 0.2 M $KHSO_4$ and 5% NaCl solutions. Drying and evaporation of solvent gives crude 3*a* (0.35 g). The sample is combined with 0.36 g of crude 3*a* from a duplicate preparation and fractionated on CC-4 silica gel (60 g) with 8.5:1.5 hexane:ethyl acetate (55 ml fractions). Fractions 10-16 give 0.326 g of 3*a*, Rf 0.58 in 1.5:1 organic layer from 9:2:5:10 ethyl acetate:acetic acid:cyclohexane: water to cyclohexane.

3b. *9-Deoxy-6,9-N-phenylimino-$\Delta^{6,8}$-13,14-dihydro-$PGI_1$*

A solution of 3*a* (0.326 g) in acetic acid (8 ml) and water (3 ml) is reacted at 40°C for 50 minutes. The purple solution is diluted with ether. Immediate color discharge is observed. The solution is washed with 5% NaCl, dried and evaporated. The residue deposits 68 mg of product compound (Rf 0.40 in 3:1 organic layer 9:2:5:10 ethyl acetate:acetic acid:cyclohexane:water to cyclohexane from ether-hexane.

IR (mull) 900, 1045, 1057, 1115, 1178, 1200, 1255, 1495, 1580, 1695, 2600 (sh), 3200 $cm^{-1}$

Example 4

9-Deoxy-6,9-N-phenylimino-$\Delta^{6,8}$-13,14-dihydro-$PGI_1$ methyl ester

A solution of 9-deoxy-6,9-N-phenylimino-$\Delta^{6,8}$-13,14-dihydro-$PGI_1$ (68 mg) is reacted with excess ethereal diazomethane. The solution is washed with 0.2 M $KHSO_4$, 5% NaCl and 5% $NaHCO_3$ solutions. Drying and evaporation of solvent gives a crystalline yellow residue. Purification on silica gel (12 g) with 3:2 hexane:ehtyl acetate (15 ml fractions) gives the product compound which deposits 25 mg, Rf 0.51 in 2:1 cyclohexane:ethyl acetate, from ether-hexane.

The compounds of the present invention are surprisingly suitable for the administration to humans and animals for the prophylactic or therapeutic treatment of allergies of a reagin or non-reagin-mediated nature. Asthma is preferably treated but any allergy wherein slow reacting substance of anaphylaxis (SRSA) is throught to be involved as a pharmacological mediator of anaphylaxis can be treated. For example, the compounds can be used for treatment of such conditions as allergic rhinitis, food allergy and urticaria as well as asthma.

An effective but essentially non-toxic quantity of the compound is employed in treatment. The dosage of the compound used in treatment depends on the route of administration and the potency of the particular compound. A dosage schedule for humans of from 0.1 to 20 mg. of compound in a single dose, administered parenterally or by inhalation in the compositions of this invention is effective for preventing or diminishing the severity of allergy attacks. More preferably, a single dose is from 0.5 to 10 mg. of compound. The amount of compound in a single orgal dose may be from 2 to 200, and is preferably from 5 to 100, mg. The dosage may be repeated up to four times daily.

For administration, it is preferred to use a composition of the invention, which comprises a compound of the invention in association with a physiologically acceptable excipient. Such compositions may be prepared in forms such as powders, granules, sterile parenteral solutions or suspensions, eyedrops, oral solutions or suspensions, and oil-in-water and water-in-oil emulsions. It will often be desirable to prepare a composition of the invention in a unit dosage form such as tablets, coated tablets, capsules, pills, powder packets, cachets, ampoules and vials. Aerosols with metered discharges may also be used.

For oral administration, either solid or fluid dosage forms can be prepared. For preparing solid compositions such as tablets, the compound of formula I is mixed with conventional ingredients such as talc, magnesium stearate, dicalcium phosphate, magnesium aluminium silicate, calcium sulphate, starch, lactose, acacia, methylcellulose or a functionally similar material, as a pharmaceutical diluent or carrier. Capsules are prepared by mixing the compound with an inert pharmaceutical diluent and filling the mixture into a hard gelatin capsule of appropriate size. Soft gelatin capsules are prepared by machine encapsulation of a slurry of the compound with an acceptable vegetable oil, light liquid petrolatum or other inert oil.

Fluid dosage forms suitable for oral administration are syrups, elixirs and suspensions. Syrups can

be prepared by dissolving the compound in an aqueous vehicle together with sugar, aromatic flavouring agents and preservatives. Elixirs may be prepared by using a hydro-alcoholic (ethanol) vehicle with suitable sweeteners such as sugar and saccharin, together with an aromatic flavouring agent. Suspensions can be prepared with an aqueous vehicle with the aid of a suspending agent such as acacia, tragacanth or methylcellulose.

For parenteral administration, fluid dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter-sterilized before filling into a suitable vial or ampoule and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the compound is suspended in the vehicle instead of being dissolved and sterilization cannot be accomplished by filtration. The compound can be sterilized by exposure to ethylene oxide or an equivalent gas before suspending in the sterile vehicle. Advantageously, a surfactant or wetting agent is included in the composition to facilitate uniform distribution of the compound.

Compositions for inhalation are of three basic types:

1) a powder mixture preferably micropulverized with particle size, preferably from about 2 to about 5 microns;

2) an aqueous solution to be sprayed with a nebulizer;

3) an aerosol with volatile propellant in a pressurized container.

The powders are quite simply prepared by mixing a compound of Formula I with a solid base which is compatible with lung tissue, preferably lactose. The powders are packaged in a device adapted to emit a measured amount of powder when inhaled thruogh the mouth.

Aqueous solutions are prepared by dissolving the compound of the Formula I in water and adding salt to provide an isotonic solution and buffering to a pH compatible with inhalation. The solutions are dispersed in a spray device or nebulizer and sprayed into the mouth while inhaling.

Aerosols are prepared by dissolving a compound of Formula I in water or ethanol and mixing with a volatile propellant and placing in a pressurized container having a metering valve to release a predetermined amount of material.

The liquefied propellant employed is one which has a boiling point below 18°C at atmospheric pressure. For use in compositions intended to produce aerosols for medicinal use the liqueified propellant should be non-toxic. Among the suitable liquefied propellants which may be employed are the lower alkanes containing up to five carbon atoms, such as butane and pentane and the lower alkyl chlorides such as methyl, ethyl and propyl chloride. Further suitable liquefied propellants are the fluorinated and fluorochlorinated lower alkanes such as are sold under the trade marks "Freon" and "Genetron." Mixtures of the above-mentioned propellants may suitably be employed. Examples of these propellants are dichlorodifluoromethane ("Freon 12"), dichlorotetrafluoroethane ("Freon 114"), trichloromonofluoromethane ("Freon 11"), dichloromonofluoromethane ("Freon 21"), monochlorodifluoromethane ("Freon 22"), trichlorotrifluoroethane ("Freon 113"), difluoroethane ("Genetron 142-A") and monochlorotrifluoromethane ("Freon 13").

The term "unit dosage form" refers to physically discrete units suitable as unitary dosages for human subjects and animals, each unit containing a predetermined quantity of active material calculated to produce the desired therapeutic effect.

## Claims

1. A compound of formula I

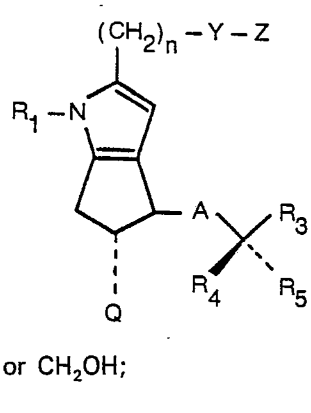

I

wherein Q is hydrogen, OH or $CH_2OH$;
*n* is 2 or 4;

Y is $—CH_2CH_2—$, *trans*-CH=CH— or $—CH_2CF_2—$;

Z is $COOR_8$, $CONR_{13}R_{14}$ or $CH_2OR_9$ in which $R_8$ is selected from hydrogen, $C_{1-6}$ alkyl, a pharmacologically acceptable metal or amine cation, phenyl, phenyl substituted by up to 3 chlorine atoms or $C_{1-3}$ alkyl radicals and *p*-substituted phenyl in which the substituent is $NHCOR_{10}$, $COR_{11}$, $OCOR_{12}$ or $CH=N—NH—CONH_2$ in which $R_{10}$ is methyl, phenyl, acetamidophenyl, benzamidophenyl or $NH_2$, $R_{11}$ is methyl, phneyl, $NH_2$ or methoxy and $R_{12}$ is phenyl or acetamidophenyl, $R_{13}$ and $R_{14}$ are indpendently selected from hydrogen, $C_{1-6}$ alkyl, phenyl and benzyl, and $R_9$ is selected from hydrogen, $C_{1-6}$ alkyl, benzoyl and acetyl;

$R_1$ is hydrogen, $C_{1-4}$ alkyl, $C_{3-7}$ cycloalkyl, phenyl, phenyl substituted by up to 3 substituents selected from $C_{1-3}$ alkyl, fluorine, chlorine, trifluoromethyl and methoxy, phenyl substituted by $COOR_8$ in which $R_8$ is as defined above, phenyl substituted by $CH_2OR_9$ in which $R_9$ is as defined above, or biphenylyl;

A is $—CH_2CH_2—$, *cis*-CH=CH—, *trans*-CH=CH— or —C≡C—;

either one of $R_4$ and $R_5$ is hydrogen or $C_{1-3}$ alkyl and the other is hydroxy, methoxy or acetoxy or $R_4$ and $R_5$ are both hydrogen or $R_4$ and $R_5$ taken together are oxo; and

$R_3$ is $CR_6R_7—X_1—CH_3$, $CR_6R_7—X_2—E$ or *cis*-$CH_2—CH=CH— CH_2—CH_3$ in which $X_1$ is $C_{1-4}$ alkylene, $X_2$ is a valence bond, O or $C_{1-6}$ alkylene, $R_6$ and $R_7$ are independently selected from hydrogen, fluorine and $C_{1-4}$ alkyl, provided that $CR_6R_7$ is not CFAlkyl and that neither $R_6$ nor $R_7$ is fluorine when $X_2$ is O, and E is phenyl, phenyl substituted by up to 3 substituents indpendently selected from $C_{1-3}$ alkyl, fluorine, chlorine, trifluoromethyl and methoxy, phenyl substituted by $COOR_8$ in which $R_8$ is as defined above, phenyl substituted by $CH_2OR_9$ in which $R_9$ is as defined above, or biphenylyl.

2. A compound as claimed in claim 1 wherein Y is $—CH_2CH_2—$ or $—CH_2CF_2—$;

Z is $COOR_8$ in which $R_8$ is hydrogen, methyl or a pharmacologically acceptable metal or amine cation;

$R_1$ is hydrogen, $C_{1-4}$ alkyl, cyclohexyl or phenyl;

A is $—CH_2CH_2—$, *trans*-CH=CH— or —C≡C—;

either one of $R_4$ and $R_5$ is hydrogen or methyl and the other is hydroxy or $R_4$ and $R_5$ are each hydrogen or $R_4$ and $R_5$ taken together are oxo;

$R_6$ and $R_7$ when $R_3$ is $CR_6R_7—X_1—CH_3$, are each fluroine or methyl and, when $R_3$ is $CR_6R_7—X_2—E$, are each hydrogen or methyl; and

E, if present, is phenyl optionally substituted by one or two substituents independently selected from fluorine, chlorine, trifluoromethyl and methoxy.

3. A compound as claimed in claim 1 wherein Q is OH; *n* is 2; Y is $—CH_2CH_2—$ or $—CH_2CF_2$; Z is COOH or $COOCH_3$; $R_1$ is hydrogen, methyl or phenyl; A is *trans*-CH=CH— or —C≡C—; $R_4$ is hydrogen; $R_5$ is hydroxy; and $R_3$ is ($C_4$ alkyl)-methyl.

4. A compound as claimed in claim 1 wherein Q is OH; *n* is 2; Y is *trans*-CH=CH—; Z is COOH, $COOCH_3$ or $CONHR_{14}$ in which $R_{14}$ is hydrogen, methyl or phenyl; $R_1$ is hydrogen or methyl; A is *trans*-CH=CH—; $R_4$ is methyl; $R_5$ is hydroxy; and $R_3$ is ($C_4$ alkyl)methyl.

5. A compound as claimed in claim 1 wherein Q is OH; *n* is 2; Y is $—CH_2CH_2—$; Z is COOH; $R_1$ is phenyl; A is *trans*-CH=CH—; $R_4$ is hydrogen; $R_5$ is hydroxy; and $R_3$ is 1,1-dimethyl-($C_4$ alkyl)methyl, phenethyl or phenoxymethyl.

6. 9-Deoxy-6,9-N-phenylimino-$\Delta^{6,8}$-prostacyclin or its methyl ester.

7. 9-Deoxy-6,9-N-phenylimino-$\Delta^{6,8}$-13,14-dihydroprostacyclin or its methyl ester.

8. A process for preparing a compound as claimed in claim 1 wherein Z is COOH, or a hydroxy-protected analogue thereof, which comprises reacting a compound of the formula

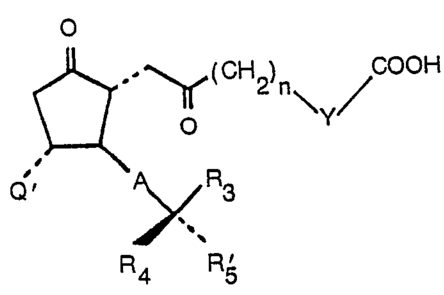

V

wherein Q′ and $R_5'$ are as defined for Q and $R_5$ in claim 1 but may also be protected OH, and *n*, Y, A, $R_3$ and $R_4$ are as defined in claim 1, with an amine of the formula

$R_1NH_2$

wherein $R_1$ is as defined in claim 1, in an organic solvent.

9. A pharmaceutical composition comprising a compound as claimed in any of claims 1 to 7 in association with a physiologically acceptable excipient.

**Revendications**

1. Composé de formule I

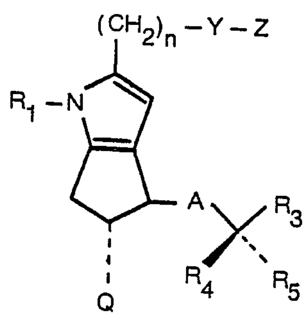

I

dans laquelle Q est l'hydrogène, le groupe OH ou un group $CH_2OH$;
*n* est égal à 2 ou 4;
Y représente $—CH_2CH_2—$, *trans*-CH=CH— ou $—CH_2CF_2—$;
Z est un groupe $COOR_8$, $CONR_{13}R_{14}$ ou $CH_2OR_9$ dans lequel $R_8$ est choisi entre l'hydrogène, un groupe alkyle en $C_1$ à $C_6$, un cation de métal ou d'amine pharmacologiquement acceptable, le groupe phényle, un groupe phényle substitué avec jusqu'à 3 atomes de chlore ou radicaux alkyle en $C_1$ à $C_3$ et un groupe phényle substitué en para dont le substituant est un groupe $NHCOR_{10}$, $COR_{11}$, $OCOR_{12}$ ou $CH=N—NH—CONH_2$, où $R_{10}$ est un radical méthyle, phényle, acétamidophényle, benzamidophényle ou $NH_2$, $R_{11}$ est un radical méthyle, phényle, $NH_2$ ou méthoxy et $R_{12}$ est un radical phényle ou acétamidophényle, $R_{13}$ et $R_{14}$ sont choisis, indépendamment, entre l'hydrogène, des radicaux alkyle en $C_1$ à $C_6$, le radical phényle et le radical benzyle, et $R_9$ est choisi entre l'hydrogène, un radical alkyle en $C_1$ à $C_6$, le radical benzoyle et le radical acétyle;
$R^1$ est l'hydrogène, un radical alkyle en $C_1$ à $C_4$, cycloalkyle en $C_3$ à $C_7$, phényle, phényle substitué avec jusqu'à 3 substituants choisis entre des radicaux alkyle en $C_1$ à $C_3$, le fluor, le chlore, le radical trifluorométhyle et le radical méthoxy, un groupe phényle substitué par un radical $COOR_8$ dans lequel $R_8$ alla définition donnée ci-dessus, un groupe phényle substitué par le radical $CH_2OR_9$ dans lequel $R_9$ a la définition donnée ci-dessus, ou un groupe biphénylyle;
A est un groupe $—CH_2CH_2—$, *cis*-CH=CH—, *trans*-CH=CH— ou —C≡C—;
l'un ou lautre de $R_4$ et $R_5$ est l'hydrogène ou un groupe alkyle en $C_1$ à $C_3$ et l'autre est un radical hydroxy, méthoxy ou acétoxy ou bien $R_4$ et $R_5$ sont tous deux de l'hydrogène ou bien $R_4$ et $R_5$ forment conjointement un radical oxo; et
$R_3$ est un groupe $CR_6R_7—X_1—CH_3$, $CR_6R_7—X_2—E$ ou *cis*-$CH_2—CH=CH—CH_2—CH_3$ où $X_1$ est un radical alkylène en $C_1$ à $C_4$, $X_2$ est une liaison de valence, O ou un radical alkylène en $C_1$ à $C_6$, $R_6$ et $R_7$ sont choisis, indépendamment, entre l'hydrogène, le fluor et un radical alkyle en $C_1$ à $C_4$, sous réserve que $CR_6R_7$ ne soit pas un groupe CF-alkyle et que ni l'un ni l'autre de $R_6$ et $R_7$ ne représente le fluor lorsque $X_2$ représente O, et E est un groupe phényle, phényle substitué avec jusqu'à 3 substituants choisis, indépendamment, entre des substituants alkyle en $C_1$ à $C_3$, fluoro, chloro, trifluorométhyle et méthoxy, un groupe phényle substitué par un radical $COOR_8$ dans lequel $R_8$ a la définition donnée ci-dessus, un groupe phényle substitué par un radical $CH_2OR_9$ dans lequel $R_9$ a la définition donnée ci-dessus, ou le groupe biphénylyle.

2. Composé suivant la revendication 1, dans lequel Y est un groupe $—CH_2CH_2—$ ou $—CH_2CF_2—$;
Z est un groupe $COOR_8$ dans lequel $R_8$ est l'hydrogène, le groupe méthyle ou un cation de métal ou d'amine pharmacologiquement acceptable;
$R_1$ est l'hydrogène, un groupe alkyle en $C_1$ à $C_4$, cyclohexyle ou phényle;
A est un groupe $—CH_2CH_2—$, *trans*-CH=CH— ou —C≡C—;
l'un de $R_4$ et $R_5$ est l'hydrogène ou le groupe méthyle et l'autre est un groupe hydroxy, ou bien $R_4$ et $R_5$ représentent chacun l'hydrogène, ou bien $R_4$ et $R_5$ forment conjointement un radical oxo;
$R_6$ et $R_7$, lorsque $R_3$ est un groupe $CR_6R_7—X_1—CH_3$, représentent chacun le fluor ou le radical méthyle et, lorsque $R_3$ est un groupe $CR_6R_7—X_2—E$, $R_6$ et $R_7$ représentent chacun l'hydrogène ou le radical méthyle; et
E, s'il est présent, représente un groupe phényle éventuallement substitué par un ou deux substituants choisis, indépendamment, entre le fluor, le chlore, le groupe trifluorométhyle et le groupe méthoxy.

3. Composé suivant la revendication 1, dans lequel Q représente OH; *n* est égal à 2; Y est un groupe $—CH_2CH_2—$ ou $—CH_2CF_2$; Z est un groupe COOH ou $COOCH_3$; $R_1$ est l'hydrogène, le groupe méthyle ou le groupe phényle; A est un groupe *trans*-CH=CH— ou —C≡C—; $R_4$ est l'hydrogène; $R_5$ est un groupe hydroxy; et $R_3$ est un groupe (alkyle en $C_4$)-méthyle.

4. Composé suivant la revendication 1, dans lequel Q représente OH; *n* est égal à 2; Y est un

groupe *trans*-CH=CH—; Z est un groupe COOH, $COOCH_3$ ou $CONHR_{14}$ dans lequel $R_{14}$ est l'hydrogène, le groupe méthyle ou le groupe phényle; $R_1$ est l'hydrogène ou le groupe méthyle; A est un groupe *trans*-CH=CH—; $R_4$ est le groupe méthyle; $R_5$ est le groupe hydroxy; et $R_3$ est un groupe (alkyle en $C_4$)-méthyle.

5. Composé suivant la revendication 1, dans lequel Q représente OH; *n* est égal à 2; Y est un groupe —$CH_2CH_2$—; Z est un groupe COOH; $R_1$ est le groupe phényle; A est le groupe *trans*-CH=CH—; $R_4$ est l'hydrogène; $R_5$ est le groupe hydroxy; et $R_3$ est un groupe, 1,1-diméthyl(alkyle en $C_4$)-méthyle, phénéthyle ou phénoxyméthyle.

6. La 9-déoxy-6,9-N-phénylimino-$\Delta^{6,8}$-prostacycline ou son ester méthylique.

7. La 9-déoxy-6,9-N-phénylimino-$\Delta^{6,8}$-13,14-dihydroprostacycline ou son ester méthylique.

8. Procédé de préparation d'un composé suivant la revendication 1, dans lequel Z représente COOH, ou un analogue à groupe hydroxy protégé de ce composé, qui consiste à faire réagir un composé de formule

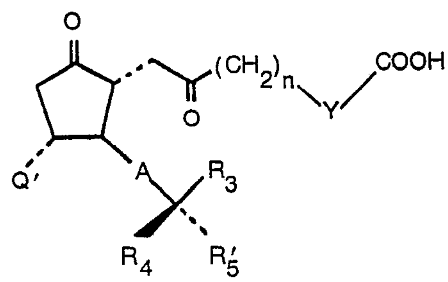

V

dans laquelle Q' et $R'_5$ ont les définitions données pour Q et $R_5$ dans la revendication 1, mais peuvent aussi représenter un groupe OH protégé, et *n*, Y, A, $R_3$ et $R_4$ ont les définitions données dans la revendication 1, avec une amine de formule

$$R_1NH_2$$

dans laquelle $R_1$ a la définition donnée dans la revendication 1, dans un solvant organique.

9. Composition pharmaceutique, comprenant un composé suivant l'une quelconque des revendications 1 à 7, en association avec un excipient physiologiquement acceptable.

**Patentansprüche**

1. Eine Verbindung der Formel I

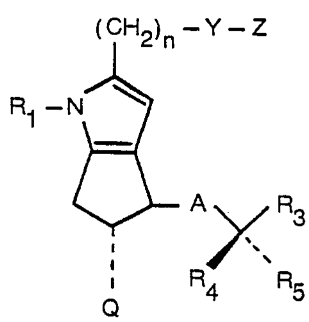

I

worin Q Wasserstoff, OH oder $CH_2OH$ ist; *n* 2 oder 4 ist; Y ist —$CH_2CH_2$—, *trans*-CH=CH— oder —$CH_2CF_2$—; z ist COOH, $CONR_{13}R_{14}$ oder $CH_2OR_9$, wobei $R_8$ von Wasserstoff, $C_{1-6}$ alkyl, einem pharmakologisch unbedenklichen Metall oder Aminkation, Phenyl, mit bis zu 3 Chloratomen oder $C_{1-3}$ Alkylresten substituiertes Phenyl und *p*-substituiertes Phenyl gewählt ist, bei dem der Substituent $NHCOR_{10}$, $COR_{11}$, $OCOR_{12}$ oder CH=N—NH—$CONH_2$ ist, wobei $R_{10}$ Methyl, Phenyl, Acedamidophenyl, Benzamidophenyl oder $NH_2$ ist, $R_{11}$ Methyl, Phenyl, $NH_2$ oder Methoxy ist und $R_{12}$ Phenyl oder Acedamidophenyl ist, $R_{13}$ und $R_{14}$ unabhängig von Wasserstoff, $C_{1-6}$Alkyl, Phenyl und Benzyl gewählt sind und $R_9$ von Wasserstoff, $C_{1-6}$Alkyl, Benzoyl und Acetyl gewählt ist;

$R_1$ ist Wasserstoff, $C_{1-4}$ Alkyl, $C_{3-7}$ Cycloalkyl, Phenyl, Phenyl welches mit bis zu 3 Substituenten substituiert ist, die von $C_{1-3}$ Alkyl, Fluor, Chlor, Trifluoromethyl und Methoxy gewählt sind, durch $COOR_8$ substituiertes Phenyl, wobei $R_8$ wie oben definiert ist, durch $CH_2OR_9$ substituiertes Phenyl, wobei $R_9$ wie oben definiert ist, oder Biphenylyl;

A ist —$CH_2CH_2$—, *cis*-CH=CH—, *trans*-CH=CH— oder —CH≡CH—;

je eins von $R_4$ und $R_5$ ist Wasserstoff oder $C_{1-3}$ Alkyl und das jeweils andere ist Hydroxy. Methoxy oder Acetoxy bzw. sind sowohl $R_4$ wie auch $R_5$ Wasserstoff oder es sind beide zusammen Oxo; und

$R_3$ ist $CR_6R_7—X_1—CH_3$, $CR_6R_7—X_2—E$ oder *cis*-$CH{=}CH—CH_2—CH_3$, worin $X_1$ ist $C_{1-4}$ Alkylen, $X_2$ ist eine Wertigkeitsbildung, O oder $C_{1-6}$ Alkylen, $R_6$ und $R_7$ sind unabhängig von Wasserstoff, Fluor und $C_{1-4}$Alkyl gewählt, vorausgesetzt, daß $CR_6R_7$ nicht CFAlkyl ist und daß weder $R_6$ noch $R_7$ Fluor ist, falls $X_2$ ein O ist; und E ist Phenyl, mit biz zu 3 Substituenten substituiertes Phenyl, wobei besagte Substituenten unabhängig von $C_{1-3}$ Alkyl, Fluor, Chlor, Trifluoromethyl und Methoxy gewählt sind, durch $COOR_8$ substituiertes Phenyl, wobei $R_8$ wie oben definiert ist, duech $CH_2OR_9$ substituiertes Phenyl, wobei $R_9$ wie oben defineiert ist, oder Biphenylyl.

2. Eine Verbindung nach Anspruch 1, worin Y ist $—CH_2CH_2—$, oder $—CH_2CF_2—$; Z ist $COOR_8$, wobei $R_8$ Wasserstoff, pharmakologisch unbedenkliches Metall oder Aminkation ist; $R_1$ ist Wasserstoff, $C_{1-4}$ Alkyl, Cyclohexyl oder Phenyl; A ist $—CH_2—$, *trans*-$CH{=}CH—$ oder $—C{\equiv}C—$; jeweils eins von $R_4$ und $R_5$ Wasserstoff und das jeweils andere Hydroxy ist oder sowohl $R_4$ und $R_5$ jeweils Wasserstoff sind oder $R_4$ und $R_5$ zusammengenommen Oxo sind;

$R_6$ und $R_7$ sind jeweils Fluor oder Methyl, falls $R_3$ ist $CR_6R_7—X_2—E$; und E, falls anwesend, ist Phenyl, welches wahlweise durch ein oder zwei, unabhängig aus Fluor, Chlor, Trifluoromethyl und Methoxy gewählten Substituenten ist.

3. Eine Verbindung nach Anspruch 1, worin Q ist OH; *n* ist 2; Y ist $—CH_2CH_2—$ oder $—CH_2CF_2—$; Z ist COOH oder $COOCH_3$;

$R_1$ ist Wasserstoff, Methyl oder Phenyl; A ist *trans*-$CH{=}CH—$ oder $—C{\equiv}C—$; $R_4$ ist Wasserstoff; $R_5$ ist Hydroxy; und $R_3$ ist ($C_4$ Alkyl) Methyl.

4. Eine Verbindung nach Anspruch 1, worin Q ist OH; *n* ist 2; Y ist *trans*-$CH{=}CH—$; Z ist COOH, $COOCH_3$ oder $CONHR_{14}$, wobei $R_{14}$ Wasserstoff, Phenyl oder Methyl ist; $R_1$ ist Wasserstoff oder Methyl; A ist *trans*-$CH{=}CH—$; $R_4$ ist Methyl; $R_5$ ist Hydroxy; und $R_3$ ist ($C_4$ Alkyl) Methyl.

5. Eine Verbindung nach Anspruch 1, worin Q ist OH; *n* ist 2; Y ist $—CH_2CH_2—$; Z ist COOH; $R_1$ ist Phenyl; A ist *trans*-$CH{=}CH—$; $R_4$ ist Wasserstoff; $R_5$ ist Hydroxy; und $R_3$ ist 1,1-Dimethyl-($C_4$ Alkyl)Methyl, Phenethyl oder Phenoxymethyl.

6. 9-Deoxy-6,9-N-Phenylimino-$\Delta^{6,8}$-Prostacyclin oder sein Methylester.

7. 9-Deoxy-6,9-N-Phenylimino-$\Delta^{6,8}$-13,14-Dihydropros*acyclin oder sein Methylester.

8. Ein Verfahren zum Herstellen einer Verbindung nach Anspruch 1, worin Z ein durch COOH oder Hydroxy geschütztes Analog davon ist, erhalten durch Reaktion einer Verbindung der Formel:

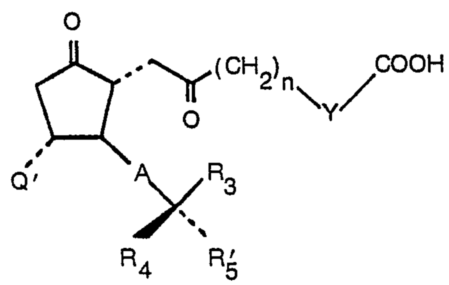

V

wobei Q′ und $R_5′$ wie Q und $R_5$ in Anspruch 1 definiert sind, mit einem Amin der Formel:

$$R_1NH_2$$

wobei $R_1$ wie in Anspruch 1 definiert ist, in einem organischen Lösungsmittel.

9. Ein pharmazeutisches Mittel, bestehend aus einer Verbindung nach einem der Ansprüche 1-7, mit einem physiologisch unbedenklichen Bindemittel.